# EUROPÄISCHE PATENTSCHRIFT

(11) **EP 2 032 245 B1**
(45) Veröffentlichungstag und Bekanntmachung des Hinweises auf die Patenterteilung: **02.11.2011**
(21) Anmeldenummer: 07724448.1
(22) Anmeldetag: 21.04.2007
(51) Int. Cl.: B01J 8/04, C07C 1/20, C07C 11/06

(54) **REAKTOR ZUR HERSTELLUNG VON C2- BIS C8- OLEFINEN AUS EINEM OXYGENAT, WASSERDAMPF UND EINEN ODER MEHRERE KOHLENWASSERSTOFFE ENTHALTENDEM STOFFSTROM**
REACTOR FOR THE PRODUCTION OF C2 TO C8 OLEFINS FROM AN OXYGENATE, WATER VAPOR, AND ONE OR MORE MATERIAL FLOWS CONTAINING HYDROCARBON
RÉACTEUR POUR LA FABRICATION D'OLÉFINES C2 À C8 A PARTIR D'UN OXYGÉNAT, DE VAPEUR D'EAU ET D'UN FLUX DE SUBSTANCES CONTENANT UN OU PLUSIEURS HYDROCARBURES

(30) Priorität: 03.06.2006 DE 102006026103
(43) Veröffentlichungstag der Anmeldung: 11.03.2009
(73) Patentinhaber: Lurgi AG, 60295 Frankfurt am Main (DE)
(72) Erfinder: BACH, Hermann, 56412 Heiligenroth (DE); BREHM, Lothar, 61138 Niederdorfelden (DE); BOHLE, Jürgen, 60596 Frankfurt am Main (DE); QUASS, Gunter, 60435 Frankfurt am Main (DE); HEINZ, Günther, 65618 Selters-Eisenbach (DE); BARTELS, Katja, 60599 Frankfurt am Main (DE); DÖRR, Heinrich, 64668 Rimbach (DE); KÖMPEL, Harald, 63263 Neu-Isenburg (DE)
(86) Internationale Anmeldenummer: PCT/EP2007/003512
(87) Internationale Veröffentlichungsnummer: WO 2007/140844

(56) Entgegenhaltungen:
- WO-A-03/086607
- DE-B- 1 258 003
- US-A- 4 542 252
- US-A- 4 544 781
- US-A1- 2006 063 956

## Beschreibung

Die Erfindung betrifft einen Reaktor zur Herstellung von C₂- bis C₈- Olefinen, vorzugsweise Propylen, aus gasförmigem Oxygenat, vorzugsweise Dimethylether (DME) und/oder Methanol (MEOH), sowie H₂O und einen oder mehrere der Kohlenwasserstoffe C₂-, C₄-, C₅-, C₆-, C₇-, C₈- Olefine und -Paraffine enthaltenden, eine Temperatur von 400 bis 470° C aufweisenden Stoffstrom, mit mehreren innerhalb eines geschlossenen, stehenden Behälters angeordneten, von dem Stoffstrom von oben nach unten durchströmten Reaktionsstufen, jeweils bestehend aus einem Tragboden mit einer darauf befindlichen, aus einer Schüttung aus körnigem Molekularsieb-Katalysator gebildeten Festbettzone. Die Erfindung bezieht sich auch auf ein Verfahren zum Betrieb des Reaktors.

Aus der DE-A-102 33 975 ist eine Vorrichtung zur Herstellung von Propylen aus MEOH bekannt. Dabei wird ein gasförmiger Einsatzstrom aus MEOH/DME und Wasser bei Betriebstemperaturen von 250 bis 460° C über mehrere, in einem vertikalen Behälter senkrecht übereinander angeordnete von oben nach unten vom Einsatzstrom durchströmte Reaktionsstufen geleitet. Die Reaktionsstufen bestehen jeweils aus einem Träger, einem darauf gelagerten Rost mit einer Maschenweite von 1/4" und einer darüber angeordneten 300 mm dicken Schicht aus SiO₂- oder Al₂O₃-Kugeln, auf der eine 750 mm dicke Schicht eines formselektiven Katalysators vom Pentasil-Typ aus zylinderförmigen Körnern mit einem Durchmesser von 1/16" und einer Länge von 1/8" aufliegt, die mit einer 200 mm dicken Schicht aus Keramikkugeln mit einem Durchmesser von 1/2" und einem darauf aufgelegten Drahtgewebe übergedeckt ist. Die Umsetzung des Einsatzstroms an den Reaktionsstufen erfolgt bei Eintrittstemperaturen von 400 bis 460°C und Drücken von 0,5 bis 3,0 bar. Zur Abkühlung des aus den Reaktionsstufen austretenden Reaktionsgemisches sind zwischen den Reaktionsstufen Wärmetauscher angeordnet, mittels denen das Reaktionsgemisch jeweils auf eine Temperatur von 400 bis < 460° C abgekühlt wird. Der Querschnitt der Eintrittsöffnungen der Wärmetauscher entspricht jeweils dem Querschnitt der Austrittsöffnungen der davor liegenden Reaktionsstufe. Der aus dem Behälter abgeleitete Produktstrom wird in eine DME, H₂O und MEOH enthaltende Flüssigphase und eine Kohlenwasserstoffe enthaltende Gasphase getrennt und aus den Kohlenwasserstoffen Propylen abgetrennt. Der Nachteil dieses Verfahrens ist darin zu sehen, dass die Reaktion in den Reaktionsstufen nicht isotherm verläuft und die Kühlung des Reaktionsgemisches mittels der zwischen den Reaktionsstufen befindlichen Wärmetauschern konstruktiv vergleichsweise aufwendig ist und strömungstechnisch ungünstig verläuft.

Es ist daher die Aufgabe der vorliegende Erfindung, den eingangs beschriebenen Reaktor so aufzubauen und das Verfahren zum Betrieb des Reaktors so zu führen, dass sich die infolge des exothermen Reaktionsverlaufs im Bereich von 400 bis 500° C liegende Temperatur des aus den einzelnen Reaktionsstufen austretende Reaktionsgemisches vor dem Eintritt in die stromabwärts nächst folgende Reaktionsstufe auf eine Temperatur von 380 bis 470° C absenken lässt. Darüber hinaus soll das Einbringen der Katalysatorschicht in die einzelnen Reaktionsstufen vereinfacht und die Durchströmen der Katalysatorschicht verbessert werden.

Gelöst ist diese Aufgabe dadurch, dass jeder Tragboden aus nebeneinander zwischenraumfrei angeordneten, fest miteinander verbundenen Zellen aufgebaut und in dem Behälter frei aufgehängt ist, die Zellen mit einer Schicht aus Molekularsieb-Katalysator gefüllt sind, jeweils in dem von zwei benachbarten Reaktionsstufen nach oben und unten begrenzten Zwischenraum ein Zerstäubersystem in Form einer Schar von Düsenrohren zum gleichmäßigen Versprühen einer DME und/oder MEOH enthaltenden, überwiegend aus H₂O bestehenden, eine Temperatur von 25 bis 150° C aufweisenden Flüssigphase mittels einer wassergesättigten, überwiegend DME und/oder MEOH enthaltenden, eine Temperatur von 170 bis 300° C aufweisenden Gasphase in Richtung auf die stromabwärts nächst folgende Reaktionsstufe vorgesehen ist. Durch das Versprühen der Flüssigphase wird die Temperatur des aus der Reaktionsstufe mit einer Temperatur von 400 bis 500° C austretenden Reaktionsgemisches auf einen Wert von 380 bis 470° C gesenkt, so dass der Reaktionsverlauf quasi isotherm ist. Die Flüssigphase kann bis zu 30 Vol. % DME und/oder MEOH und die Gasphase bis zu 80 Vol. % DME und bis zu 30 Vol. % MEOH enthalten.

Als Molekularsieb-Katalysatoren kommen insbesondere synthetische Zeolithe verschiedenen Typs, beispielsweise ZSM-5, Pentasil, MFI-Z oder MeAPSO zum Einsatz.

Die Körner des Molekularsieb-Katalysators sind vorzugsweise zylinderförmig und besitzen eine mittlere Länge von 3,5 bis 7,0 mm oder 2,1 bis 4,5 mm und einen mittleren Durchmesser von 3,1 bis 3,4 mm oder 3,3 bis 3,7 mm.

Im Rahmen der besonderen Ausgestaltung der Erfindung beträgt die Schichtdicke des in die Zellen des Tragbodens eingefüllten Molekularsieb-Katalysators 100 bis 1000 mm und nimmt stromabwärts von einer Reaktionsstufe zur nächst folgenden Reaktionsstufe stetig zu, wobei zweckmäßigerweise die Schichtdicker der ersten Reaktionsstufe 100 bis 500 mm und diejenige der letzten Reaktionsstufe 500 bis 1000 mm beträgt. Durch diese Maßnahme und durch die Anpassung der Stoffstrommengen ist eine für die exotherm verlaufende Umsetzung konstante Verweilzeit des Reaktionsgemisches in allen Reaktionsstufen gewährleistet.

Der aus zahlreichen Zellen, vorzugsweise in der Gestalt von Quadern, Würfeln oder geraden, gleichseitigen Prismen aufgebaute Tragboden besitzt eine ausreichende Biegungssteifigkeit gegenüber dem aus dem Eigengewicht, dem Gewicht der Schicht aus Molekularsieb-Katalysator und dem Gewicht der inerten Kugeln gebildeten Gesamtgewicht. Die Größe der Durchbiegung des Tragbodens ist vernachlässigbar und eine freie Aufhängung des Tragbodens in dem Behälter gewährleistet.

Damit der Molekularsieb-Katalysator und darunter liegende 100 bis 400 mm dicke Schicht aus inerten Kugeln nicht aus den Zellen herausfallen können, besitzt der Tragboden bodenseitig eine drahtgewebe-, streckmetall-, lochblechartige oder dergleichen gestaltete Überdeckung. Der Durchmesser der inerten Kugeln beträgt im unteren Schichtabschnitt 10 bis 15 mm und im oberen Schichtabschnitt 5 bis 8 mm. Ein solcher Schichtaufbau verhindert eine Ausbildung von Strömungskanälen, durch das Reaktionsgemisch vorzeitig aus der Reaktionsstufe austreten kann.

Deckflächenseitig besitzt der Tragboden ebenfalls eine drahtgewebe-, streckmetall-, lochblechartige oder dergleichen ausgebildete Überdeckung, auf der eine 50 bis 200 mm dicke Schicht aus inerten Kugeln mit einem Durchmesser von 5 bis 15 mm aufgelegt ist. Durch die Anordnung dieser Schicht ist gewährleistet, dass die in den jeweils von zwei benachbarten Reaktionsstufen gebildeten Zwischenraum versprühte Flüssigphase sicher und vollständig verdampft wird.

Nach einer besonderen Ausgestaltung der Erfindung besteht das Zerstäubersystem jeweils aus einer Schar von Düsenrohren mit in regelmäßigen Abständen angeordneten Zweistoffdüsen mit Außenmischung, vorzugsweise mit Vollkegelcharakteristik mit einem Strahlwinkel von 15 bis 35° C. Durch die äußere Vermischung von Gas- und Flüssigphase lässt sich der Durchsatz der überwiegend H₂O und DME und/oder MEOH enthaltenden, eine Temperatur von 25 bis 150° C aufweisender Flüssigphase und der wassergesättigten, überwiegend DME und MEOH enthaltenden, eine Temperatur von 170 bis 300° C aufweisenden Gasphase unabhängig voneinander einstellen.

Bei einer Ausführungsform der Erfindung besteht das jeweils zwischen zwei benachbarten Reaktionsstufen angebrachte Zerstäubersystem aus jeweils von beiden Seiten einer die Behälterachse einschließenden Senkrechtebene von der Peripherie des Behälters her horizontal eingeführten, spiegelbildlich angeordneten, an den in Strömungsrichtung liegenden Enden geschlossenen Düsenrohren, die jeweils senkrecht zu der die Behälterachse einschließenden senkrechten Ebene in regelmäßigem Abstand parallel zueinander verlegt sind und der Abstand der Düsenrohrenden zu der die Behälterachse einschließenden Ebene 20 bis 500 mm beträgt. Durch diese Anordnung wird erreicht, dass die freie Weglänge der Flüssigkeit begrenzt wird.

Eine optimale Kühlwirkung durch die versprühte Flüssigphase lässt sich dann erzielen, wenn bei würfel- oder quaderförmiger Gestaltung der den Tragboden bildenden Zellen das Düsenrohr jeweils von auf einer Geraden liegenden Doppel- oder Einzelzellwand den gleichen Abstand besitzt und in der diese Zellwand einschließenden senkrechten Ebene verlaufend angeordnet ist.

In Sonderfällen besteht die Möglichkeit, die Düsenrohre in Form von Kreisevolventen zu verlegen.

Zum Betrieb des Reaktors wird ein gasförmiges Oxygenat, vorzugsweise DME und/oder MEOH, sowie H₂O enthaltender, eine Temperatur von 150 bis 300° C aufweisender Prozessstrom auf eine Temperatur von 100 bis 160° C abgekühlt, in eine Flüssigphase und eine Gasphase getrennt und Flüssigphase und Gasphase in mehrere Teilströme, deren Anzahl jeweils der Anzahl der zwischen den Reaktionsstufen bestehenden Zwischenräume entspricht, aufgeteilt. Es werden, bezogen auf einen Zwischenraum, jeweils ein Gasphasenteilstrom nach einer Erwärmung auf eine Temperatur von 170 bis 300° C und ein Flüssigphasenteilstrom nach einer Abkühlung auf eine Temperatur von 25 bis 150° C einem Zerstäuber zugeführt und in den Zwischenraum versprüht. Durch diese Maßnahmen lässt sich die Eintrittstemperatur des aus der Reaktionsstufe in den Zwischenraum austretenden Reaktionsgemisches vor dem Eintritt in die nächst folgende Reaktionsstufe auf die gewünschte Temperatur einregeln.

Um eine vollständige Verdampfung der Flüssigphase zu erreichen, ist es angebracht, die Flüssigphase mittels der Gasphase zu einem feinen Tropfenspektrum mit einem Durchmesser von 10 bis 100 µm zu zerreißen.

Die Erfindung ist nachstehend durch ein Ausführungsbeispiel und in den Zeichnungen näher und beispielhaft erläutert.

Es zeigen:
- Fig. 1: ein Verfahrensfließbild mit schematisch dargestelltem Reaktor
- Fig. 2: eine Draufsicht auf einen aus quaderförmigen Zellen aufgebauten Tragboden
- Fig. 3: eine Draufsicht auf einen aus wabenförmigen Zellen aufgebauten Tragboden
- Fig. 4: eine Draufsicht auf einen in einen Behälter eingebauten aus quaderförmigen Zellen gebildeten Tragboden mit darüber angeordneten Düsenrohren
- Fig. 5: eine Draufsicht auf den Tragboden gemäß Fig. 4 mit den Grundfläche der von den eingebauten Zweistoffdüsen erzeugten Sprühkegeln
- Fig. 6: eine Draufsicht auf die in Form von Kreisevolventen verlegte Schar von Zerstäubern mit den Grundflächen der von den eingebauten Zweistoffdüsen erzeugten Sprühkegeln
- Fig. 7: einen vergrößerten Ausschnitt (X) aus Fig. 1

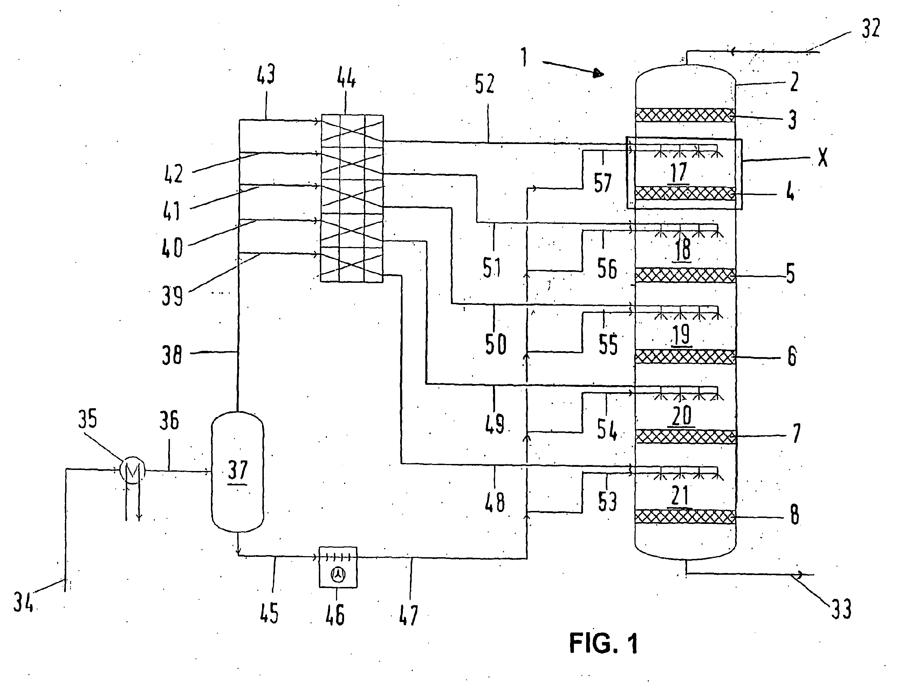

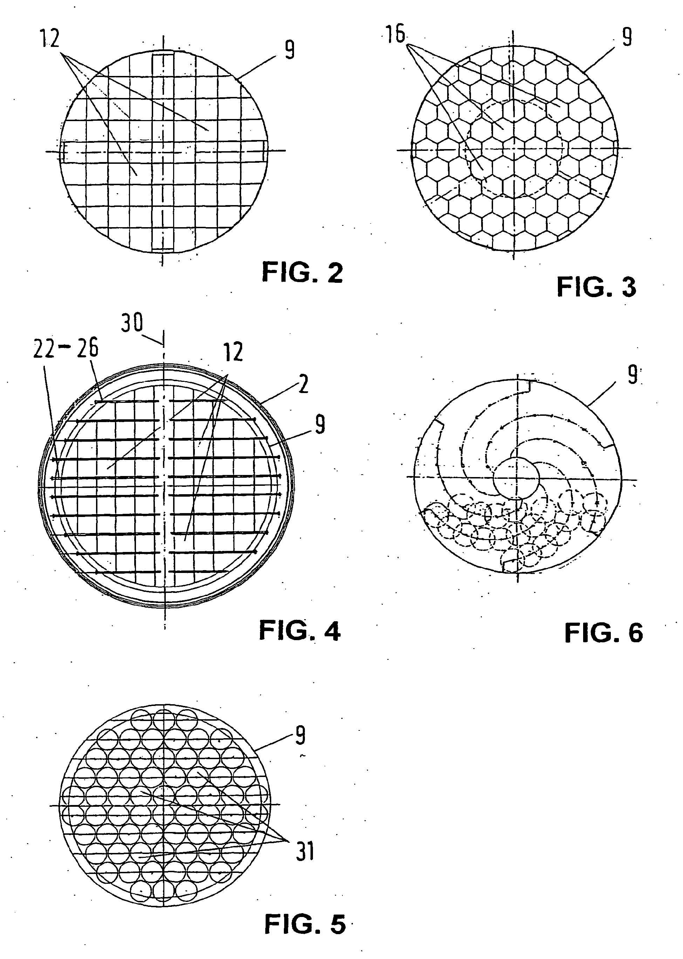

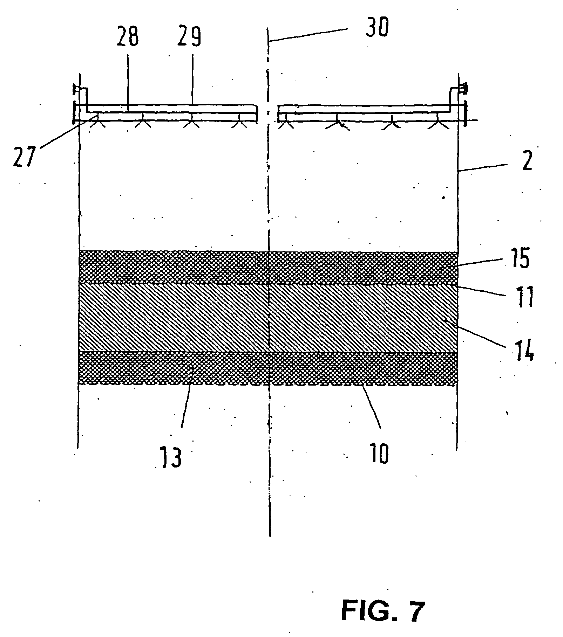

Gemäß Fig. 1 besteht der Reaktor (1) aus einem senkrechten, zylindrischen, geschlossenen Behälter (2) mit sechs untereinander eingebauten Reaktionsstufen (3, 4, 5, 6, 7, 8), die im einzelnen, wie in Fig. 2 bis Fig. 5 und dem in Fig. 7 wiedergegebenen Ausschnitt (X) aus Fig. 1 dargestellt, aus einem frei-tragenden Tragboden (9) aus quaderförmigen, grund- und deckflächenseitig mit einem Drahtgewebe (10, 11) überdeckten, zwischenraumfrei fest miteinander verbundenen Zellen (12) aufgebaut sind. Der untere Abschnitt der Zellen (12) ist mit einer 100 mm dicken Schicht (13) aus keramischen Kugeln, deren Durchmesser in der unteren Schichthälfte 0,5" und in der oberen Schichthälfte 0,25" beträgt, gefüllt. Auf diese Kugelschicht (13) ist eine Schicht (14) aus körnigem formselektiven Zeolith-Katalysator vom Pentasiltyp aufgeschüttet. Auf dem deckflächenseitig angeordneten Drahtgewebe (11) befindet sich eine 100 ± 10 mm dicke Schicht (15) aus keramischen Kugeln mit einem Durchmesser von 0,25". Vom Kopf des Behälters nimmt stromabwärts die Dicke der Katalysatorschicht (14) von Reaktionsstufe zu Reaktionsstufe (3, 4, 5, 6, 7, 8) stetig zu. Bei der stromabwärts ersten Reaktionsstufe (3) beträgt die Schichtdicke 253 ± 10 mm, der zweiten Reaktionsstufe (4) 286 ± 10mm, der dritten Reaktionsstufe (5) 327 ± 10 mm, der vierten Reaktionsstufe (6) 384 ± 10 mm, der fünften Reaktionsstufe (7) 462 ± 10 mm und der sechsten Reaktionsstufe (8) 588 ± 10 mm. Fig. 3 zeigt einen aus wabenförmigen Zellen (16) aufgebauten Tragboden (9). Die Schichtdicken sind variabel.

In den von den Reaktionsstufen (3, 4, 5, 6, 7, 8) begrenzten Zwischenräumen (17, 18, 19, 20, 21) ist jeweils eine Schar von Düsenrohren (22, 23, 24, 25, 26) mit in regelmäßigen Abständen darin eingesetzten Zweistoffdüsen (27) mit Außenmischung und Vollkegelcharakteristik mit einem Strahlwinkel von 30° verlegt. Die Zweistoffdüsen (27) zerstäuben die über das Rohr (28) zuströmende überwiegend aus Wasser bestehende, DME und MEOH enthaltende, eine Temperatur von 93° C aufweisende Flüssigphase unter Einwirkung der über das Rohr (29) zugeleiteten, eine durchschnittliche Temperatur von 175 °C besitzende wassergesättigten, DME und MEOH enthaltende Gasphase. Die Zweistoffdüsen (27) ermöglichen ein gleichmäßiges Versprühen der Flüssigphase in Form von Aerosolen. Die durch das Versprühen erzeugten großen spezifischen Oberflächen der Flüssigphase ermöglichen einen schnellen Wärme- und Stoffaustausch zwischen den Aerosolen einerseits und dem aus den Reaktionsstufen (3, 4, 5, 6, 7) austretenden Reaktionsgemisch andererseits.

Wie in Fig. 4 dargestellt, besteht das jeweils in dem von zwei benachbarten Reaktionsstufen (3, 4, 5, 6, 7, 8) begrenzten Zwischenraum (17, 18, 19, 20, 21) angebrachte Zerstäubersystem aus jeweils von beiden Seiten einer die Behälterachse einschließenden Senkrechtebene (30) von der Peripherie des Behälters (1) her in einer Horizontalebene eingeführten, spiegelbildlich angeordneten, an den in Strömungsrichtung liegenden Enden geschlossenen Düsenrohren (22, 23, 24, 25, 26), die senkrecht zu der die Behälterachse einschließenden Senkrechtebene (30) in regelmäßigem Abstand parallel zueinander verlegt sind. Die einzelnen Düsenrohre (22, 23, 24, 25, 26) besitzen jeweils von den auf einer Geraden liegenden Wänden der Zellen (12) den gleichen Abstand und sind in den die Zellwände einschließenden senkrechten Ebenen verlaufend angeordnet.

Fig. 5 zeigt die Grundflächen (31) der von den Zweistoffdüsen der Düsenrohre (22, 23, 24, 25, 26) erzeugten Vollkegel.

Für besondere Anwendungsfälle besteht gemäß Fig. 6 die Möglichkeit, die Düsenrohre in Form von Kreisevolventen zu verlegen.

Beim Betrieb des Reaktors wird dem Behälter (2) am Kopf über Leitung (32) ein eine Temperatur von 469°C aufweisender, gasförmiger Stoffstrom von 104199 kg/h, im wesentlichen bestehend aus 1819 kg MEOH, 3721 kg DME, 41841 kg Wasser und 56753 kg Kohlenwasserstoffen, aufgegeben. Aus dem Sumpf des Behälters (2) wird über Leitung (33) ein eine Temperatur von 480°C besitzender, gasförmiger Stoffstrom von 155405 kg/h, im wesentlichen bestehend aus 443 kg MEOH, 217 kg DME, 72450 kg Wasser und 79879 kg Kohlenwasserstoffen, abgezogen.

Über Leitung (34) wird ein Einsatzstrom von 51207 kg/h, der 9724 kg MEOH, 29266 kg DME und 12200 kg Wasser enthält und eine Temperatur von 156°C besitzt, in einen Kondensationsapparat (35) geleitet. Der auf eine Temperatur von durchschnittlich 150°C abgekühlte Einsatzstrom wird über Leitung (36) einem Zweiphasen-Abscheider (37) zugeführt, in dem eine Trennung in einen Gasphasestrom von 44832 kg/h, im einzelnen bestehend aus 8497 kg MEOH, 29229 kg DME und 7089 kg Wasser und einen Flüssigphasestrom von 6375 kg/h, der aus 1227 kg MEOH, 38 kg DME und 5110 kg Wasser zusammengesetzt ist, erfolgt. Der über Leitung (38) abgeführte Gasphasestrom wird in fünf Teilströme aufgeteilt, die jeweils über eine der Leitungen (39, 40, 41, 42, 43) in Platten-Wärmeaustauscher (44) strömen und in diesem auf eine Temperatur von 176°C erwärmt werden. Der über Leitung (45) den Zweiphasen-Abscheider (37) verlassende Flüssigphasestrom wird in einem Wärmeaustauscher (46) auf eine Temperatur von 93° C abgekühlt, über Leitung (47) abgeleitet und in fünf Teilströme getrennt. Von den aus den Platten-Wärmeaustauscher (44) über die Leitungen (48, 49, 50, 51, 52) abgeführten Gasphase-Teilströmen wird jeweils ein Gasphase-Teilstrom und von den über die Leitungen (53, 54, 55, 56, 57) abgeführten Flüssigphase-Teilströmen jeweils ein Flüssigphase-Teilstrom je einem Düsenrohr (22, 23, 24, 25, 26) zugeführt und die Flüssigphase unter Einwirkung der Gasphase gleichmäßig in Richtung der stromabwärts nächstfolgende Reaktionsstufe (4, 5, 6, 7, 8) in Form von Aerosolen versprüht. Durch einen zusätzlichen dem Wärmeaustauscher (46) unmittelbar nachgeschalteten, nicht dargestellten Wärmeaustauscher lässt sich die Temperatur des Flüssigphasestroms bis auf 25° C absenken.

Im einzelnen werden der über Leitung (57) zugeführten Flüssigphase-Teilstrom von 800 kg/h, im wesentlichen enthaltend 171 kg MEOH, 5 kg DME, 713 kg H₂O, und der über Leitung (52) zugeführte Gasphase-Teilstrom von 6259 kg/h, im wesentlichen enthaltend 1186 kg MEOH, 4080 kg DME und 990 kg H₂O, dem Düsenrohr (22) zugeleitet und in den zwischen der stromwärts ersten und zweiten Reaktionsstufe (3, 4) vorhandenen Zwischenraum (17) versprüht. Der über Leitung (56) aufgegebene Flüssigphase-Teilstrom von 1019 kg/h, im wesentlichen bestehend aus 196 kg MEOH, 6 kg DME und 817 kg H₂O, und der über Leitung (51) zugeführte Gasphase-Teilstrom von 7167 kg/h, im wesentlichen enthaltend 1358 kg MEOH, 4673 kg DME und 1133 kg H₂O, werden dem Düsenrohr (23) zugeleitet und die Flüssigphase in den zwischen der zweiten und dritten Reaktionsstufe (4, 5) befindlichen Zwischenraum (18) versprüht. In den von der dritten und vierten Reaktionsstufe (5, 6) begrenzten Zwischenraum (19) wird der über Leitung (55) zugeführte Flüssigphase-Teilstrom von 1197 kg/h, im wesentlichen enthaltend 230 kg MEOH, 7 kg DME und 959 kg H₂O, von dem über Leitung (50) zugeführte Gasphase-Teilstrom von 8417 kg/h, im wesentlichen enthaltend 1595 kg MEOH, 5488 kg DME und 1331 kg H₂O, versprüht. Der über Leitung (54) dem Düsenrohr (25) zugeführte Flüssigphase-Teilstrom von 1438 kg/h, im wesentlichen enthaltend 277 kg MEOH, 9 kg DME und 1153 kg H₂O. wird mittels des über Leitung (49) dem Düsenrohr (26) zugeführten Gasphase-Teilstroms von 10111 kg/h, wesentlich bestehend aus 1926 kg MEOH, 6592 kg DME und 1599 kg H₂O, in den von der vierten und fünften Reaktionsstufe (6, 7) begrenzten Zwischenraum (20) versprüht. In den von der fünften und sechsten Reaktionsstufe (7, 8) gebildeten Zwischenraum (21) wird der über Leitung (53) dem Düsenrohr (26) zugeführte Flüssigphase-Teilstrom von 1831 kg/h, im wesentlichen enthaltend 351 kg MEOH, 11 kg DME und 1468 kg H₂O, mittels des über Leitung (48) dem Zerstäuber (27) zugeführten Gasphase-Teilstroms von 12879 kg/h, im wesentlichen enthaltend 2441 kg MEOH, 8397 kg DME und 2037 kg H₂O versprüht.

Die eingesetzten Zweistoffdüsen besitzen eine Außenmischung, d.h. Gas- und Flüssigphasenteilströme treten jeweils getrennt ein und treffen sich unmittelbar am Düsenaustritt. Die Gasphase trifft dabei auf den aus dem Flüssighasen-Mundstück austretenden Vollstrahl außerhalb der Düse und reißt diesen zu einem feinen Tropfenspektrum auf. Die Regelung der Gasphase-Teilströme hat nahezu keinen Einfluss auf die Volumina der Flüssigphase-Teilströme. Das gilt auch für die Änderung der Flüssigphase-Volumenteilströme mit Wirkung auf die Gasphase-Teilströme.

## Patentansprüche

1. Reaktor zur Herstellung von C₂- bis C₈-Olefinen, vorzugsweise Propylen, aus gasförmigem Oxygenat, vorzugsweise Dimethylether (DME) und/oder Methanol (MEOH), sowie H₂O und einen oder mehrere der Kohlenwasserstoffe C₂,- C₄-. C₅-, C₆-, C₇-, C₈-Olefine und Paraffine enthaltenden, eine Temperatur von 400 bis 470° C aufweisenden Stoffstrom, mit mehreren innerhalb eines geschlossenen, stehenden Behälters (2) angeordneten, von dem von oben kommenden Stoffstrom durchströmten Reaktionsstufen (3, 4, 5, 6, 7, 8), jeweils bestehend aus einem Tragboden (9) mit einer darauf befindlichen, aus einer Schicht (14) aus körnigem Molekularsieb-Katalysator gebildeten Festbettzone, **dadurch gekennzeichnet, dass** jeder Tragboden (9) aus nebeneinander zwischenraumfrei angeordneten, fest miteinander verbundenen Zellen (12, 16) aufgebaut und in dem Behälter (2) frei aufgehängt ist, die Zellen mit einer Schicht (14) aus Molekularsieb-Katalysator gefüllt sind, jeweils in dem von zwei benachbarten Reaktionsstufen (3, 4, 5, 6, 7, 8) nach oben und unten begrenzten Zwischenraum (17, 18, 19, 20, 21) ein Zerstäubersystem in Form einer Schar von Düsenrohren (22, 23, 24, 25, 26) zum gleichmäßigen Versprühen einer DME und/oder MEOH enthaltenden, überwiegend aus H₂O bestehenden, eine Temperatur von 25 bis 150° C aufweisenden Flüssigphase mittels einer wassergesättigten, überwiegend DME und/oder MEOH enthaltenden, eine Temperatur von 170 bis 300° C aufweisenden Gasphase in Richtung auf die stromabwärts folgende Reaktionsstufe vorgesehen ist.

2. Reaktor nach Anspruch 1, **dadurch gekennzeichnet, dass** der Molekularsieb-Katalysator ein synthetischer Zeolith, beispielsweise vom ZSM-5-Typ, Pentasil-Typ, MFI-Z-Typ oder MeAPSO-Typ ist.

3. Reaktor nach einem der Ansprüche 1 und 2, **dadurch gekennzeichnet, dass** die Körner des Molekularsieb-Katalysators zylinderförmig sind und eine durchschnittliche Länge von 3,5 bis 7,0 mm oder 2,1 bis 4,5 mm und einen durchschnittlichen Durchmesser von 3,1 bis 3,4 mm oder 3,3 bis 3,7 mm besitzen.

4. Reaktor nach einem der Ansprüche 1 bis 3, **dadurch gekennzeichnet, dass** die Dicke der Schicht (14) des in den Zellen (12, 16) befindlichen Molekularsieb-Katalysators 100 bis 1000 mm beträgt und stromabwärts von einer Reaktionsstufe (3, 4, 5, 6, 7, 8) zur nächst folgenden Reaktionsstufe stetig zunimmt.

5. Reaktor nach Anspruch 4, **dadurch gekennzeichnet, dass** die Dicke der Schicht (14) des in den Zellen (12, 16) befindlichen Molekularsieb-Katalysators der ersten Reaktionsstufe (3) 100 bis 500 mm und der stromabwärts letzten Reaktionsstufe (8) 500 bis 1000 mm beträgt.

6. Reaktor nach einem der Ansprüche 1 bis 5, **dadurch gekennzeichnet, dass** die den Tragboden (9) bildenden Zellen (12, 16) die Form eines Quaders, eines Würfels oder eines geraden gleichseitigen Prismas besitzen.

7. Reaktor nach einem der Ansprüche 1 bis 5, **dadurch gekennzeichnet, dass** der Tragboden (9) boden- und deckflächenseitig eine drahtgewebe-, streckmetall-, lochblechartige oder dergleichen gestaltete Überdeckung (10, 11) aufweist.

8. Reaktor nach einem der Ansprüche 1 bis 6, **dadurch gekennzeichnet, dass** sich in den Zellen (12, 16) des Tragbodens (9) eine 50 bis 400 mm dicke Schicht (13) inerter Kugeln, deren Durchmesser im unteren Abschnitt der Schicht 10 bis 15 mm und im oberen Abschnitt 5 bis 8 mm beträgt, befindet, auf die die Schicht (14) aus Molekularsieb-Katalysator aufgeschüttet ist.

9. Reaktor nach einem der Ansprüche 1 bis 7, **dadurch gekennzeichnet, dass** auf die deckflächenseitige Überdeckung (11) des Tragbodens (9) eine 50 bis 200 mm dicke Schicht (15) inerter Kugeln mit einem Durchmesser von 5 bis 15 mm aufgetragen ist.

10. Reaktor nach einem der Ansprüche 1 bis 8, **dadurch gekennzeichnet, dass** die Düsenrohre (22, 23, 24, 25, 26) in regelmäßigen Abständen angeordnete Zweistoffdüsen mit Außenmischung, vorzugsweise mit Vollkegelcharakteristik mit einem Strahlwinkel von 15 bis 35 °, besitzen.

11. Reaktor nach einem der Ansprüche 1 bis 9, **dadurch gekennzeichnet, dass** das Zerstäubersystem aus jeweils *von* beiden Seiten einer die Behälterachse einschließenden Senkrechtebene (30) von der Peripherie des Behälters (1) her eingeführten, spiegelbildlich angeordneten, an den in Strömungsrichtung legenden Enden geschlossenen Düsenrohren (22, 23, 24, 25, 26) besteht, die jeweils senkrecht zu der die Behälterachse einschließenden senkrechten Ebene in regelmäßigen Abständen verlegt sind und der Abstand der Düsenrohrenden zu der die Behälterachse einschließenden senkrechten Ebene 20 bis 500 mm beträgt.

12. Reaktor nach Anspruch 10, **dadurch gekennzeichnet, dass** bei würfel- oder quaderförmiger Gestaltung der den Tragboden (9) bildenden Zellen (12) das einzelne Düsenrohr (22, 23, 24, 25, 26) jeweils von auf einer Geraden liegenden Zellwänden den gleichen Abstand besitzt und in der die Zellwände einschließenden senkrechte Ebene verlaufend angeordnet sind.

13. Reaktor nach einem der Ansprüche 9 und 10, **dadurch gekennzeichnet, dass** die jeweils eine Schar bildenden Düsenrohre in Form von Kreisevolventen verlegt sind.

14. Verfahren zum Betrieb des Reaktors (1) zur Herstellung von C₂- bis C₈-Olefinen, vorzugsweise Propylen, aus einem gasförmiges Oxygenat, vorzugsweise DME und/oder MEOH, sowie H₂O und einen oder mehrere der Kohlenwasserstoffe C₂-, C₄-, C₅-, C₆-, C₇, C₈- Olefine und Paraffine enthaltendem, eine Temperatur von 400 bis 470° C aufweisenden Stoffstrom, mit mehreren innerhalb eines geschlossenen, stehenden Behälters (2) angeordneten, von dem von oben kommenden Stoffstrom durchströmten Reaktionsstufen (3, 4, 5, 6, 7, 8), jeweils bestehend aus einem Tragboden (9) mit einer auf diesem befindlichen, aus einer Schicht (14) aus körnigem Molekularsieb-Katalysator gebildeten Festbettzone, wobei jeder Tragboden aus nebeneinander zwischenraumfrei angeordneten, fest miteinander verbundenen Zellen (12, 16) aufgebaut und in dem Behälter frei aufgehängt ist, die Zellen mit der Schicht aus Molekularsieb-Katalysator gefüllt sind, jeweils in dem von zwei benachbarten Reaktionsstufen nach oben und unten begrenzten Zwischenraum (17, 18, 19, 20, 21)ein Zerstäubersystem in Form einer Schar von Düsenrohren (22, 23, 24, 25, 26) zum gleichmäßigen Versprühen einer überwiegend H₂O enthaltenden, eine Temperatur von 50 bis 150° C aufweisenden Flüssigphase mittels einer wassergesättigten, überwiegend DME und/oder MEOH enthaltenden, eine Temperatur von 170 bis 300° C aufweisenden Gasphase in Richtung auf die jeweils stromabwärts folgende Reaktionsstufe vorgesehen ist, **dadurch gekennzeichnet, dass** ein gasförmiges Oxygenat, vorzugsweise DME und MEOH, sowie H₂O enthaltender, eine Temperatur von 150 bis 300° C besitzender Einsatzstrom auf eine Temperatur von 100 bis 150° C abgekühlt und in eine Flüssigphase und eine Gasphase getrennt, die Gas- und Flüssigphase in mehrere Teilströme, deren Anzahl jeweils der Anzahl der zwischen den Reaktionsstufen (3, 4, 5, 6, 7, 8) bestehenden Zwischenräumen (17, 18, 19, 20 ,21) entspricht, aufgeteilt und der einzelne Gasphase-Teifstrom mit jeweils einem Flüssigphase-Teilstrom einem Düsenrohr (22, 23, 24, 25, 26) aufgegeben und die Flüssigphase mittels der Gasphase in den korrespondierenden Zwischenraum versprüht wird.

15. Verfahren nach Anspruch 13, **dadurch gekennzeichnet, dass** die Flüssigphase mittels der Gasphase zu einem feinen Tropfenspektrum mit einem Durchmesser von 10 bis 100 µm zerrissen wird.

## Claims

1. A reactor for the production of C₂ to C₈ olefins, preferably propylene, from a material flow containing gaseous oxygenate, preferably dimethyl ether (DME) and/or methanol (MEOH), as well as H₂O and one or more of the hydrocarbons C₂-, C₄-, C₅-, C₆-, C₇-, C₈- olefins and -paraffins, which has a temperature of 400 to 470°C, with a plurality of reaction stages (3, 4, 5, 6, 7, 8) arranged inside a closed, upright container (2) and traversed by the material flow coming from above, each consisting of a supporting tray (9) with a fixed-bed zone disposed thereon and formed of a layer (14) of granular molecular sieve catalyst, **characterized in that** each supporting tray (9) is constructed of cells (12, 16) firmly connected with each other, which are arranged one beside the other without leaving a gap, and is freely suspended in the container (2), the cells are filled with a layer (14) of molecular sieve catalyst, in the space (17, 18, 19, 20, 21) defined at the top and at the bottom by two adjacent reaction stages (3, 4, 5, 6, 7, 8) an atomizer system in the form of a group of nozzle tubes (22, 23, 24, 25, 26) each is provided for uniformly spraying a liquid phase containing DME and/or MEOH, which chiefly consists of H₂O and has a temperature of 25 to 150°C, by means of a water-saturated gas phase chiefly containing DME and/or MEOH, which has a temperature of 170 to 300°C, in direction of the succeeding downstream reaction stage.

2. The reactor according to claim 1, **characterized in that** the molecular sieve catalyst is a synthetic zeolite, for example of the ZSM-5 type, pentasil type, MFI-Z type or MeAPSO type.

3. The reactor according to any of claims 1 and 2, **characterized in that** the grains of the molecular sieve catalyst are cylindrical and have an average length of 3.5 to 7.0 mm or 2.1 to 4.5 mm and an average diameter of 3.1 to 3.4 mm or 3.3 to 3.7 mm.

4. The reactor according to any of claims 1 to 3, **characterized in that** the thickness of the layer (14) of the molecular sieve catalyst present in the cells (12, 16) is 100 to 1000 mm and continuously increases in downstream direction from one reaction stage (3, 4, 5, 6, 7, 8) to the next reaction stage.

5. The reactor according to claim 4, **characterized in that** the thickness of the layer (14) of the molecular sieve catalyst of the first reaction stage (3), which is present in the cells (12, 16), is 100 to 500 mm and is 500 to 1000 mm downstream of the last reaction stage (8).

6. The reactor according to any of claims 1 to 5, **characterized in that** the cells (12, 16) forming the supporting tray (9) have the shape of a cuboid, a cube or a straight equilateral prism.

7. The reactor according to any of claims 1 to 5, **characterized in that** on its bottom and top surfaces the supporting tray (9) includes a cover (10, 11) designed in the manner of a wire mesh, expanded metal, perforated sheet or the like.

8. The reactor according to any of claims 1 to 6, **characterized in that** in the cells (12, 16) of the supporting tray (9) a 50 to 400 mm thick layer (13) of inert balls is provided, whose diameter is 10 to 15 mm in the lower portion of the layer and 5 to 8 mm in the upper portion, on which the layer (14) of molecular sieve catalyst is heaped up.

9. The reactor according to any of claims 1 to 7, **characterized in that** on the top-side cover (11) of the supporting tray (9) a 50 to 200 mm thick layer (15) of inert balls with a diameter of 5 to 15 mm is applied.

10. The reactor according to any of claims 1 to 8, **characterized in that** the nozzle tubes (22, 23, 24, 25, 26) have two-fluid nozzles arranged at regular intervals with exernal mixing, preferably with full-cone characteristic with a jet angle of 15° to 35°.

11. The reactor according to any of claims 1 to 9, **characterized in that** the atomizer system consists of nozzle tubes (22, 23, 24, 25, 26) each introduced from the periphery of the container (1) from both sides of a vertical plane (30) including the container axis, arranged mirror-symmetrically and closed at the ends lying in flow direction, which each are installed at regular intervals vertical to the vertical plane including the container axis and the distance of the nozzle tube ends to the vertical plane including the container axis is 20 to 500 mm.

12. The reactor according to claim 10, **characterized in that** with a cube-shaped or cuboid-shaped design of the cells (12) forming the supporting tray (9) the individual nozzle tube (22, 23, 24, 25, 26) each has the same distance from cell walls lying on a straight line and is arranged to extend in the vertical plane including the cell walls.

13. The reactor according to any of claims 9 and 10, **characterized in that** the nozzle tubes each forming a group are installed in the form of circular involutes.

14. A process for operating the reactor (1) for the production of C₂ to C₈ olefins, preferably propylene, from a material flow containing gaseous oxygenate, preferably DME and/or MEOH, as well as H₂O and one or more of the hydrocarbons C₂-, C₄-, C₅-, C₆-, C₇-, C₈-olefins and paraffins, which has a temperature of 400 to 470°C, with a plurality of reaction stages (3, 4, 5, 6, 7, 8) arranged inside a closed, upright container (2) and traversed by the material flow coming from above, each consisting of a supporting tray (9) with a fixed-bed zone disposed thereon and formed of a layer (14) of granular molecular sieve catalyst, wherein each supporting tray is constructed of cells (12, 16) firmly connected with each other, which are arranged one beside the other without leaving a gap, and is freely suspended in the container, the cells are filled with the layer of molecular sieve catalyst, in the space (17, 18, 19, 20, 21) defined at the top and at the bottom by two adjacent reaction stages an atomizer system in the form of a group of nozzle tubes (22, 23, 24, 25, 26) each is provided for uniformly spraying a liquid phase chiefly containing H₂O, which has a temperature of 50 to 150°C, by means of a water-saturated gas phase chiefly containing DME and/or MEOH, which has a temperature of 170 to 300°C, in direction of the succeeding downstream reaction stage, **characterized in that** a feed stream containing gaseous oxygenate, preferably DME and MEO, as well as H₂O, which has a temperature of 150 to 300°C, is cooled to a temperature of 100 to 150°C and is separated into a liquid phase and a gas phase, the gas and liquid phases are divided into a plurality of partial streams, whose number each corresponds to the number of spaces (17, 18, 19, 20, 21) existing between the reaction stages (3, 4, 5, 6, 7, 8), and the individual gas phase partial stream is charged to a nozzle tube (22, 23, 24, 25, 26) with one liquid phase partial stream each and the liquid phase is sprayed into the corresponding space by means of the gas phase.

15. The process according to claim 13, **characterized in that** the liquid phase is disrupted to a fine droplet formation with a diameter of 10 to 100 µm by means of the gas phase.

## Revendications

1. Réacteur de production d'oléfines en C₂ à C₈, de préférence de propylène, à partir d'un produit oxygéné gazeux, de préférence à partir d'oxyde de diméthyle (DME) et/ou de méthanol (MEOH), ainsi que d'un courant de matière contenant H₂O et un ou plusieurs des hydrocarbures oléfines et paraffines en C₂, C₄, C₅, C₆, C₇, C₈ et ayant une température de 400 à 470°C, comprenant plusieurs étages (3, 4, 5, 6, 7, 8) de réaction disposés dans une colonne (2) fermée, parcourue par le courant de matière venant du haut et constituée respectivement d'un plateau (9) support ayant une zone de lit fixe se trouvant dessus et formée d'une couche (14) de catalyseur en tamis moléculaire en grain, **caractérisé en ce que** chaque plateau (9) support est constitué de cellules (12, 16) disposées côte à côte sans espace intermédiaire et reliées solidement entre elles et est suspendu librement dans la colonne (2), les cellules sont garnies d'une couche (14) de catalyseur en tamis moléculaire respectivement dans l'espace (17, 18, 19, 20, 21) intermédiaire délimité vers le haut ou vers le bas par deux étages (3, 4, 5, 6, 7, 8) de réaction voisins, un système d'atomisation sous la forme d'un assemblage de lances (22, 23, 24, 25, 26) est prévu pour la pulvérisation uniforme d'une phase liquide contenant du DME et/ou du MEOH, constituée de manière prépondérante d'H₂O et ayant une température de 25 à 150°C, au moyen d'une phase gazeuse saturée en eau contenant de manière prépondérante du DME et/ou du MEOH et ayant une température de 170 à 300°C en direction de l'étage de réaction suivant en aval.

2. Réacteur suivant la revendication 1, **caractérisé en ce que** le catalyseur en tamis moléculaire est un zéolithe synthétique, par exemple du type ZSM-5, du type pentasil, du type MFI-Z ou du type MeAPSO.

3. Réacteur suivant l'une des revendications 1 et 2, **caractérisé en ce que** les grains du catalyseur en tamis moléculaire sont cylindriques et ont une longueur moyenne de 3,5 à 7,0 mm ou de 2,1 à 4,5 mm et un diamètre moyen de 3,1 à à 3,4 mm ou de 3,3 à 3,7 mm.

4. Réacteur suivant l'une des revendications 1 à 3, **caractérisé en ce que** l'épaisseur de la couche (11) du catalyseur en tamis moléculaire se trouvant dans les cellules (12, 16) va de 100 à 1 000 mm et augmente vers l'aval d'un étage (3, 4, 5, 6, 7, 8) de réaction à l'étage de réaction immédiatement suivant.

5. Réacteur suivant 1a revendication 4, **caractérisé en ce que** l'épaisseur de la couche (14) du catalyseur en tamis moléculaire se trouvant dans les cellules (12, 16) du premier étage (3) de réaction va de 100 à 500 mm et du dernier étage (8) de réaction en aval va de 500 à 1 000 mm.

6. Réacteur suivant l'une des revendications 1 à 5, **caractérisé en ce que** les cellules (12, 16) formant le plateau (9) support ont la forme d'un parallélépipède, d'un cube ou d'un prisme (3) équilatéral.

7. Réacteur suivant l'une des revendications 1 à 5, **caractérisé en ce que** le plateau (9) support a du côté de la surface du fond et du sommet un recouvrement (10, 11) en toile métallique, en métal déployé du type à tôle perforée ou conformé d'une manière analogue.

8. Réacteur suivant l'une des revendications 1 à 6, **caractérisé en ce qu'**une couche (13) de billes inertes d'une épaisseur de 50 à 400 mm, dont le diamètre des billes est, dans la partie inférieure de la couche, de 10 à 15 mm et, dans la partie supérieure, de 5 à 8 mm, se trouve dans les cellules (12, 16) du plateau (9) support et est répandue sur la couche (14) de catalyseur en tamis moléculaire.

9. Réacteur suivant l'une des revendications 1 à 7, **caractérisé en ce que**, sur le recouvrement (11) du plateau (9) support du côté de la surface de sommet, est déposée une couche (15) d'une épaisseur de 50 à 200 mm de billes inertes ayant un diamètre de 5 à 15 mm.

10. Réacteur suivant l'une des revendications 1 à 8, **caractérisé en ce que** les lances (22, 23, 24, 25, 26) ont des buses à deux matières disposées à intervalles réguliers avec mélange extérieur ayant, de préférence, une caractéristique de cône plein ayant un angle de faisceau de 15 à 35°.

11. Réacteur suivant l'une des revendications 1 à 9, **caractérisé en ce que** le système d'atomisation est constitué de lances (22, 23, 24, 25, 26), qui sont introduites à partir de la périphérie de la colonne (1), respectivement des deux côtés d'un plan (30) vertical passant par l'axe de la colonne, disposées symétriquement comme en un miroir, qui sont fermées aux extrémités se trouvant dans la direction d'écoulement et qui sont mises à des intervalles réguliers, respectivement perpendiculairement au plan vertical passant par l'axe de la colonne, et la distance des extrémités des lances au plan vertical, passant par l'axe de la colonne va de 20 à 500 mm.

12. Réacteur suivant la revendication 10, **caractérisé en ce que**, pour une conformation en cube ou parallélépipédique des cellules (12) formant le plateau (9) support, la lance (22, 23, 24, 25, 26) individuelle est à la même distance des parois de cellules se trouvant sur une droite et elles sont disposées dans le plan perpendiculaire passant par les parois de cellules.

13. Réacteur suivant l'une des revendications 9 et 10, **caractérisé en ce que**, respectivement, des lances formant un assemblage sont mises sous la forme d'une développante de cercle.

14. Procédé pour faire fonctionner le réacteur (1) de production d'oléfines en C₂ à C₈, de préférence de propylène, à partir d'un courant de matière contenant un produit oxygéné gazeux, de préférence du DME et/ou du MEOH, ainsi que de l'H₂O et un ou plusieurs des hydrocarbures oléfines et paraffines en C₂, C₄, C₅, C₆, C₇, C₈ et ayant une température de 400 à 470°C, comprenant plusieurs étages (3, 4, 5, 6, 7, 8) de réaction disposés à l'intérieur d'une colonne (2) fermée, parcourue par le courant de matière venant du haut et constituée respectivement d'un plateau (9) support ayant une zone de lit fixe se trouvant sur celui-ci et formée d'une couche (14) de catalyseur en tamis moléculaire en grain, chaque plateau étant formé de cellules (12, 16) disposées les unes à côté des autres sans espace intermédiaire et reliées fixement entre elles et étant suspendu librement dans la colonne, les cellules étant garnies de la couche (14) de catalyseur en tamis moléculaire, respectivement dans l'espace (17, 18, 19, 20, 21) intermédiaire délimité vers le haut et vers le bas par ceux étages (3, 4, 5, 6, 7, 8) de réfaction voisins, un système d'atomisation sous la forme d'un assemblage de lances (22, 23, 24, 25, 26) étant prévu pour la pulvérisation uniforme d'une phase liquide contenant de manière prépondérante de l'H₂O et ayant une température de 50 à 150°C au moyen d'une phase gazeuse saturée en eau contenant de manière prépondérante du DME et/ou du MEOH et ayant une température de 170 à 300°C en direction de l'étage de réaction suivant en aval respectivement, **caractérisé en ce que** l'on refroidit jusqu'à une température de 100 à 150°C un courant de charge contenant du produit oxygéné gazeux, de préférence du DME et du MEOH ainsi que de l'H₂O, et ayant une température de 150 à 300°C et on le sépare en une phase liquide en une phase gazeuse, on subdivise la phase gazeuse et la phase liquide en plusieurs courants partiels don't le nombre correspond respectivement au nombre des espaces (17, 18, 19, 20, 21) intermédiaire constitués entre les étages (3, 4, 5, 6, 7, 8) de réaction et on charge le courant partiel en phase gazeuse, avec respectivement un courant partiel en phase liquide, dans une lance (22, 23, 24, 25, 26) et ou pulvérise la phase liquide au moyen de la phase gazeuse dans l'espace intermédiaire correspondant.

15. Procédé suivant la revendication 14, **caractérisé en ce que** l'on rompt la phase liquide au moyen de la phase gazeuse en un spectre fin de gouttelettes d'un diamètre de 10 à 100 µm.
